# EUROPÄISCHE PATENTSCHRIFT

(11) **EP 3 162 807 B1**
(45) Veröffentlichungstag und Bekanntmachung des Hinweises auf die Patenterteilung: **14.02.2018**
(21) Anmeldenummer: 15192085.7
(22) Anmeldetag: 29.10.2015
(51) Int. Cl.: C07F 7/18, C08F 20/00, C09D 175/00

(54) **MONOALLOPHANATE AUF BASIS VON ALKOXYSILANALKYLISOCYANATEN**
MONOALLOPHANATES BASED ON ALKOXYSILANALKYL ISOCYANATES
MONOALLOPHANATES A BASE D'ALCOXYSILANE ALKYLISOCYANATES

(43) Veröffentlichungstag der Anmeldung: 03.05.2017
(73) Patentinhaber: Evonik Degussa GmbH, 45128 Essen (DE)
(72) Erfinder: SPYROU, Emmanouil, 46514 Schermbeck (DE); KRECZINSKI, Manfred, 44652 Herne (DE); UNKELHÄUSSER, Tobias, 48249 Dülmen (DE); NAUMANN, Sabine, 44651 Herne (DE); LOESCH, Holger, 44627 Herne (DE); EWALD, Marion, 45768 Marl (DE); WEIHRAUCH, Thomas, 48249 Dülmen (DE)

(56) Entgegenhaltungen:
- WO-A1-2015/007588
- DE-A1-102005 009 790
- DE-A1-102005 041 954
- JP-A- 2015 101 716

## Beschreibung

Die Erfindung betrifft neue Monoallophanate auf Basis von Alkoxysilanalkylisocyanaten ein Verfahren zur Herstellung und die Verwendung.

Polyurethane haben sich seit vielen Jahrzehnten als hochwertige Bausteine für Lack-, Klebstoff-, Dichtstoff und Kunststoffsysteme erwiesen. Dabei können zusätzliche Alkoxysilangruppen hier eine wichtige Rolle z.B. in Hinblick auf Netzwerkdichte, Chemikalienbeständigkeit und Kratzfestigkeit spielen, in erste Linie durch die Ausbildung von Siloxanstrukturen. Auch solche Systeme sind bekannt.

Moleküle die sowohl über Alkoxysilangruppen verfügen, als auch Isocyanatgruppen aufweisen bieten die Möglichkeit beide genannte Funktionalitäten, Siloxane und Polyurethangruppen durch eine Komponente einzufügen. Auch solche Substanzen sind schon lange in Gebrauch, z.B. Isocyanatoalkyltrialkoxysilane. Aus diesen Substanzen können durch Umsetzung mit Polyalkoholen isocyanatfreie feuchtigkeitshärtende Vernetzer hergestellt werden. Grundsätzlich können solche "SiPUR" (alkoxysilanhaltige Polyurethane) mit weiteren Isocyanaten zu Allophanaten umgesetzt werden, um bestimmte Materialeigenschaften zu modifizieren, z.B. die Viskosität.

So beschreibt die DE102005041953A1 eine Umsetzung eines Polyols mit einem mittleren Molekulargewicht von 3000 - 20000 g/Mol mit einem Überschuss von Isocyanatopropyltrimethoxysilan, so dass es nach der Polyurethanbildung zu einer Allophanatreaktion kommt.

In DE102005041954A1 wird ein Polyurethan aus einem Polyol und einem Diisocyananat (z.B. IPDI, Isophorondiisocyananat) mit Isocyanatopropyltrimethoxysilan versetzt und so lange erhitzt bis sich Allophanatstrukturen ausbilden.

EP2089444A1 beansprucht allophanathaltige Polyurethane, die neben einer ungesättigten Funktionalität noch Silangruppen haltige Komponenten aufweist.

Ähnlich sieht es auch in EP2089445A1 aus, hier müssen diese Polyurethane aber noch zusätzlich eine dispergieraktive Komponente enthalten, um sie wassergängig zu machen.

Auch in der US20140097392A1 ist von allophanat- und alkoxysilanhaltigen Polymeren die Rede, die in diesem Fall mit einem Farbstoff abgemischt werden.

J. Kozakiewicz et al. publizierten in Progress in Organic Coatings 72 (2011) 120- 130 silanhaltige Blockierungsmittel für Polyisocyanate, die über eine Allophanatgruppe eingebracht wurden. Offensichtlich gibt es einen Bedarf für weitere Alkoxysilangruppen tragende Verbindungen vor allem für Kratzfestanwendungen. Alle bisherigen Beispiele haben aber gemein, dass es sich hier immer um Polymere und/oder Polyurethane handelt, die sowohl hohe Viskositäten aufweisen als auch schwierig zu reinigen bzw. zu isolieren sind.

JP 2015 101716 A beschreibt die Verwendung einer einzelnen spezifischen Verbindung mit Allophanatstruktur zur Oberflächenbehandlung.

DE 10 2005 009790 A1 beschreibt die Verwendung einer einzelnen spezifischen Verbindung mit Allophanatstruktur als Klebstoffzusatz.

DE 10 2005 041954 A1 beschreibt die Verwendung von Polyallophanaten für Beschichtungen und Klebstoffe.

Aufgabe dieser Erfindung war es, neue Alkoxysilangruppen tragende Verbindungen mit Kratzfesteigenschaften zugänglich zu machen, die nicht den Nachteil des Standes der Technik haben, insbesondere aber einfach herzustellen sind, niedrige Viskositäten aufweisen und mit wenig Aufwand zu reinigen sind.

Überraschend wurde gefunden, dass Monoallophanate bestehend aus einem Isocyanat modifzierten Alkoxysilangruppen haltigen Monourethan die gewünschten Eigenschaften hat.

Gegenstand der Erfindung sind demnach Monoallophanate mit der Formel 1: 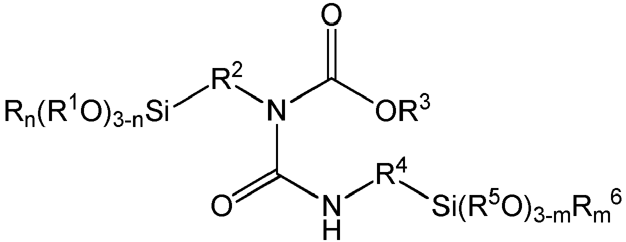 wobei R, R¹-R⁶ unabhängig voneinander gleiche oder verschiedene Kohlenwasserstoffreste mit 1-8 C-Atomen bedeuten, wobei diese verzweigt oder cyclisch sein können, und m und n gleich 0 bedeutet. Die Variablen m und n stehen dabei für 0. R¹ und R⁵ sind unabhängig voneinander bevorzugt Methyl oder Ethyl.

R² und R⁴ sind bevorzugt unabhängig voneinander Methyl oder Propyl.

Besonders bevorzugt ist R¹ = R⁵ und R² = R⁴. Bevorzugt sind R¹ und R⁵ gleich Methyl oder Ethyl, und R² und R⁴ Methyl oder Propyl. Ganz besonders bevorzugt sind R¹ = R⁵ = Methyl und R² = R⁴ = Propyl.

Gegenstand ist auch ein Verfahren zur Herstellung der erfindungsgemäßen Monoallophanate durch eine Reaktion von
A) einem Alkoxysilangruppen haltigen Isocyanat mit
B) einem Alkoxysilangruppen haltigen Monourethan, bei Temperaturen im Bereich von 20 bis 220 °C.

Alkoxysilangruppen haltige Isocyanate A) weisen die Formel 2 auf:

R⁶m (OR⁵)₃₋ₘSi-R⁴-NCO

wobei R⁶, R⁵ und R⁴ unabhängig voneinander gleiche oder verschiedene Kohlenwasserstoffreste mit 1-8 C-Atomen bedeuten, wobei diese verzweigt oder cyclisch sein können, und m gleich 0 bedeutet. Die Variable m ist 0. R⁴ ist bevorzugt Methyl oder Propyl.

R⁵ ist bevorzugt Methyl oder Ethyl.

Bevorzugt sind Verbindungen mit R⁴ gleich Methyl oder Propyl, und R⁵ gleich Methyl oder Ethyl.

Besonders bevorzugt ist Isocyanatopropyltrimethoxysilan.

Alkoxysilangruppen haltige Monourethane B) weisen die Formel 3 auf:

Rₙ (OR¹)₃₋ₙ Si-R²-NH-(C=O)-OR³

wobei R, R¹, R² und R³ unabhängig voneinander gleiche oder verschiedene Kohlenwasserstoffreste mit 1-8 C-Atomen bedeuten, wobei diese verzweigt oder cyclisch sein können, und n gleich 0 bedeutet.

R¹ ist bevorzugt Methyl oder Ethyl.

R² ist bevorzugt Methyl oder Propyl.

Bevorzugt ist R³ = R¹.

Bevorzugt sind Verbindungen mit R² gleich Methyl oder Propyl, und R¹ gleich Methyl oder Ethyl und R³ = R¹.

Besonders bevorzugt ist N-Trimethoxysilylpropylmethylcarbamat.

Die Herstellung der erfindungsgemäßen Monoallophanate erfolgt im Allgemeinen lösungsmittelfrei oder unter Verwendung von nicht-protischen Lösemitteln, wobei die Umsetzung diskontinuierlich oder kontinuierlich erfolgen kann. Die Reaktion wird durchgeführt in geeigneten Aggregaten, z.B. Rührkessel, Extruder, Statikmischern, Knetkammern. Die Reaktion kann bei Raumtemperatur, das heißt bei Temperaturen im Bereich von 20 bis 22 °C, geführt werden, bevorzugt werden jedoch höhere Temperaturen im Bereich 80 bis 220 °C, insbesondere im Bereich von 80 bis 120 °C verwendet. Zur Beschleunigung der Reaktion können vorteilhaft in der Urethanchemie bekannte Katalysatoren, z.B. Metallorganische Verbindungen, wie Zinn oder Zink haltige Verbindungen, Salze, wie z.B. Zn(II)chlorid und/oder Basen verwendet werden. Geeignet sind zum Beispiel Sn-, Bi-, Zn- und andere Metallcarboxylate wie z,B. Dibutylzinndilaurat, Zinnoctoat, Bismuthneodecanoat, tert.-Amine wie beispielsweise 1,4-Diazabicyclo[2.2.2]octan (DABCO), Triethylamin, Amidine und Guanidine, sowie quarternäre Ammoniumsalze, bevorzugt Tetralkylammoniumsalze und/oder quarternäre Phosphoniumsalze .

Als Katalysatoren kommen Metallacetylacetonate in Frage. Beispiele dafür sind Zinkacetylacetonat, Lithiumacetylacetonat Eisenacetylacetonat und Zinnacetylacetonat, allein oder in Mischungen. Bevorzugt wird Zinkacetylacetonat eingesetzt. Als Katalysatoren kommen außerdem quarternäre Ammoniumacetylacetonate oder quarternäre Phosphoniumacetylacetonate in Frage.

Die Reaktion wird unter Ausschluss von Wasser durchgeführt. Bevorzugt wird die Reaktion lösemittelfrei durchgeführt.

Gegenstand der Erfindung ist auch die Verwendung der erfindungsgemäßen Monoallophanate in Lackzusammensetzungen für Metall-, Kunststoff-, Glas-, Holz-, MDF- (Middle Density Fiber Boards) oder Ledersubstrate oder sonstigen hitzeresistenten Untergründen.

Gegenstand der Erfindung ist auch die Verwendung der erfindungsgemäßen Monoallophanate in Klebstoffzusammensetzungen für Verklebungen von Metall-, Kunststoff-, Glas-, Holz-, MDF- oder Ledersubstraten oder sonstigen hitzeresistenten Untergründen.

Die vorliegende Erfindung wird weiterhin durch die folgenden nicht beschränkenden Beispiele veranschaulicht, denen weitere Merkmale, Ausführungsformen, Aspekte und Vorteile der vorliegenden Erfindung entnommen werden können.

### Beispiele

1) Herstellung der erfindungsgemäßen Monoallophanate
   a) 236 g (1 mol) Trimethoxysilylpropylmethylcarbamat (Evonik Industries AG) und 205 g Isocyanatopropyltrimethoxysilan (Evonik Industries AG) werden miteinander vermischt und 2 h auf 175 °C erhitzt. Danach werden die Ausgangsprodukte bei 100 °C und 0,3 mbar über eine Kurzweg-Destillation abgetrennt. Es verbleiben 183 g (41,5%) einer reinen, wasserklaren Flüssigkeit. C13-NMR in CDCI₃ (ppm): 156,9 (1); 154,4 (1); 53,5 (1); 50,5 (10); 46,3 (1); 43,2 (1); 23,2 (1); 22,6 (1), 6,7 (1); 6,5 (1).
      Viskosität beträgt ca. 200 mPas und ist damit sehr niedrigviskos.
   b) 236 g (1 mol) Trimethoxysilylpropylmethylcarbamat (Evonik Industries AG), 205 g Isocyanatopropyltrimethoxysilan (Evonik Industries AG) und 0,4 g Zinn(II)chlorid werden miteinander vermischt und 1 h auf 150 °C erhitzt. Danach werden die Ausgangsprodukte bei 90 °C und 0,3 mbar über eine Kurzweg-Destillation abgetrennt. Es verbleiben 339 g (76,9%) einer reinen, wasserklaren Flüssigkeit. (NMR s.o.)
   c) 236 g (1 mol) Trimethoxysilylpropylmethylcarbamat (Evonik Industries AG), 205 g Isocyanatopropyltrimethoxysilan (Evonik Industries AG) und 1 g Eisen(III)acetylacetonat werden miteinander vermischt und 3 h auf 90 °C erhitzt. Danach werden die Ausgangsprodukte bei 90 °C und 0,3 mbar über eine Kurzweg-Destillation abgetrennt. Es verbleiben 184 g (41,5%) einer reinen, wasserklaren Flüssigkeit. (NMR s.o.)
2) Lack-Formulierung:
   44,5 g Setalux 1760 (OH-funktionelles Acrylat, Nuplex industries) und 30 g vom erfindungsgemäßen Produkt a) werden mit 25 g Butylacetat/Xylol (1:1) gemischt und mit 0,5 g Katalysator (VESTANAT EP-CAT 11, Evonik Industries AG) versetzt. Diese Mischung wird mit einem 120 µm Spiralrakel auf ein Stahlblech aufgerakelt und 22 min bei 140 °C eingebrannt. Die Beschichtung (30 µm Schichtdicke) weist eine Pendelhärte von 176 s und eine Chemikalienbeständigkeit von >150 MEK Doppelhübe auf. Sie ist also vollständig ausgehärtet.
   Kratzfestigkeit:
   Der Ausgangsglanzgrad beträgt 86 Skalenteile (Skt.) (20°). Nach dem Bürstentest ist der Glanzgrad auf 84 Skt. gesunken. Der Crockmetertest führt zu einem Glanzgrad von 83 Skt. Der Glanzgradverlust beträgt also 2 bzw. 3 Skt. und damit ist die Kratzfestigkeit exzellent! Wertung: Glanzgradverlust durch Kratzer 0-9 Skt. exzellent, 10-20 sehr gut, 21-34 gut, 35-44 mittel, >45 schlecht
      Im Vergleich dazu ergeben handelsübliche 2K-PUR Lacke auf Basis von Setalux 1760 einen Glanzgradverlust von ca. 30 Skt.
   Bürstentest (nass):
   Gerät: U 1 Fabr.-Nr. 003, Hersteller: BASF L + F, Baujahr:1993
      Die Lackoberfläche wird mit einem Siebgewebe (Nylon-Siebgewebe Nr. 11, Maschenweite 25 µm), welches mit einer Masse (2 kg) belastet wird, geschädigt. Das Siebgewebe und die Lackoberfläche werden mit einer Waschmittellösung (0,25 %ige Persil-Lösung in Wasser) reichlich benetzt. Die Prüftafel wird mittels eines Motorantriebes in Hubbewegungen unter dem Siebgewebe vor- und zurückgeschoben. Vor und nach dem Test wird der Glanzgrad gemessen.

### Crockmetertest (trocken)

Gerät: U 1 Fabr.-Nr. 003, Hersteller: BASF L + F, Baujahr:1993
Die Lackoberfläche wird mit einem Gewebe (3M 281Q WetODry Polishing Paper), welches mit einer Masse (920 g) belastet wird, geschädigt. Die Prüftafel wird mittels eines Motorantriebes in Hubbewegungen unter dem Gewebe vor- und zurückgeschoben.

## Patentansprüche

1. Monoallophanate mit der Formel 1: 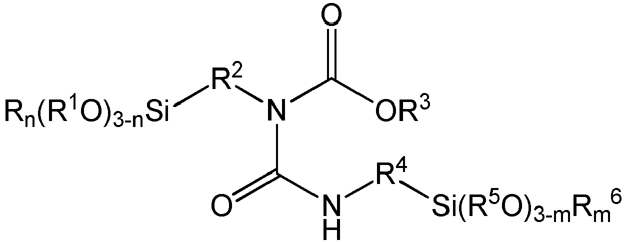 wobei R, R¹-R⁶ unabhängig voneinander gleiche oder verschiedene Kohlenwasserstoffreste mit 1-8 C-Atomen bedeuten, wobei diese verzweigt oder cyclisch sein können, und m und n gleich 0 bedeutet.

2. Monoallophanate mit der Formel 1 nach Anspruch 1, wobei R¹ und R⁵ unabhängig voneinander Methyl oder Ethyl bedeuten.

3. Monoallophanate mit der Formel 1 nach Anspruch 1, wobei R² und R⁴ unabhängig voneinander Methyl oder Propyl bedeuten.

4. Monoallophanate mit der Formel 1 nach Anspruch 1, wobei R¹ = R⁵ und R² = R⁴ bedeuten.

5. Monoallophanate mit der Formel 1 nach Anspruch 1, wobei m und n gleich 0, R¹ und R⁵ gleich Methyl oder Ethyl, und R² und R⁴ gleich Methyl oder Propyl bedeuten.

6. Monoallophanate mit der Formel 1 nach Anspruch 1, wobei m und n gleich 0, R¹ = R⁵ = Methyl und R² = R⁴ = Propyl bedeuten.

7. Verfahren zur Herstellung der Monoallophanate nach Anspruch 1-6 durch eine Reaktion von
A) einem Alkoxysilangruppen haltigen Isocyanat mit
B) einem Alkoxysilangruppen haltigen Monourethan, bei Temperaturen im Bereich von 20 bis 220 °C.

8. Verfahren zur Herstellung der Monoallophanate nach Anspruch 7 wobei die Alkoxysilangruppen haltigen Isocyanate A) die Formel 2 aufweisen:
R⁶m (OR⁵)₃₋ₘSi-R⁴-NCO
wobei R⁶, R⁵ und R⁴ unabhängig voneinander gleiche oder verschiedene Kohlenwasserstoffreste mit 1-8 C-Atomen bedeuten, wobei diese verzweigt oder cyclisch sein können, und m gleich 0 bedeutet.

9. Verfahren zur Herstellung der Monoallophanate nach Anspruch 8 wobei m gleich 0 und R⁴ gleich Methyl oder Propyl, und R⁵ gleich Methyl oder Ethyl bedeuten.

10. Verfahren zur Herstellung der Monoallophanate nach Anspruch 8 wobei Isocyanatopropyltrimethoxysilan eingesetzt wird.

11. Verfahren zur Herstellung der Monoallophanate nach Anspruch 7 wobei die Alkoxysilangruppen haltige Monourethane B) die Formel 3 aufweisen:
Rₙ (OR¹)₃₋ₙ Si-R²-NH-(C=O)-OR³
wobei R, R¹, R² und R³ unabhängig voneinander gleiche oder verschiedene Kohlenwasserstoffreste mit 1-8 C-Atomen bedeuten, wobei diese verzweigt oder cyclisch sein können, und n gleich 0 bedeutet.

12. Verfahren zur Herstellung der Monoallophanate nach Anspruch 11 wobei n gleich 0 und R² gleich Methyl oder Propyl, und R¹ gleich Methyl oder Ethyl und R³ = R¹ bedeuten.

13. Verfahren zur Herstellung der Monoallophanate nach Anspruch 11 wobei N-Trimethoxysilylpropylmethylcarbamat eingesetzt wird.

14. Verwendung der Monoallophanate nach Anspruch 1-6 in Lackzusammensetzungen und Klebstoffzusammensetzungen.

## Claims

1. Monoallophanates having the formula 1: 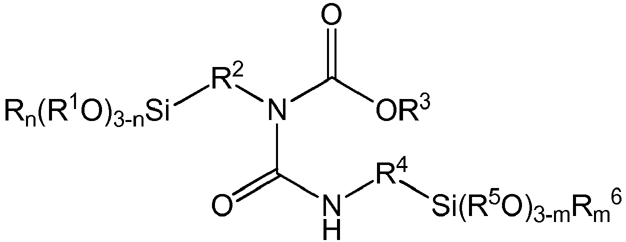 where R, R¹-R⁶ are each independently identical or different hydrocarbyl radicals having 1-8 carbon atoms, which may be branched or cyclic, and m and n are each 0.

2. Monoallophanates having the formula 1 according to Claim 1, where R¹ and R⁵ are each independently methyl or ethyl.

3. Monoallophanates having the formula 1 according to Claim 1, where R² and R⁴ are each independently methyl or propyl.

4. Monoallophanates having the formula 1 according to Claim 1, where R¹ = R⁵ and R² = R⁴.

5. Monoallophanates having the formula 1 according to Claim 1, where m and n are each 0, R¹ and R⁵ are each methyl or ethyl and R² and R⁴ are each methyl or propyl.

6. Monoallophanates having the formula 1 according to Claim 1, where m and n are each 0, R¹ = R⁵ = methyl and R² = R⁴ = propyl.

7. Process for preparing the monoallophanates according to Claims 1-6, by a reaction of
A) an isocyanate containing alkoxysilane groups with
B) a monourethane containing alkoxysilane groups, at temperatures in the range from 20 to 220°C.

8. Process for preparing the monoallophanates according to Claim 7,
wherein the isocyanates A) containing alkoxysilane groups have the formula 2:
R⁶m (OR⁵)₃₋ₘSi-R⁴-NCO
where R⁶, R⁵ and R⁴ are each independently identical or different hydrocarbyl radicals having 1-8 carbon atoms, which may be branched or cyclic, and m is 0.

9. Process for preparing the monoallophanates according to Claim 8,
where m is 0 and R⁴ is methyl or propyl, and R⁵ is methyl or ethyl.

10. Process for preparing the monoallophanates according to Claim 8, wherein isocyanatopropyltrimethoxysilane is used.

11. Process for preparing the monoallophanates according to Claim 7,
wherein the monourethanes B) containing alkoxysilane groups have the formula 3:
Rₙ (OR¹)₃₋ₙ Si-R²-NH-(C=O)-OR³
where R, R¹, R² and R³ are each independently identical or different hydrocarbyl radicals having 1-8 carbon atoms, which may be branched or cyclic, and n is 0.

12. Process for preparing the monoallophanates according to Claim 11, where n is 0 and R² is methyl or propyl, and R¹ is methyl or ethyl and R³ = R¹.

13. Process for preparing the monoallophanates according to Claim 11, wherein N-trimethoxysilylpropylmethyl carbamate is used.

14. Use of the monoallophanates according to Claims 1-6 in paint compositions and adhesive compositions.

## Revendications

1. Monoallophanates de formule 1 : 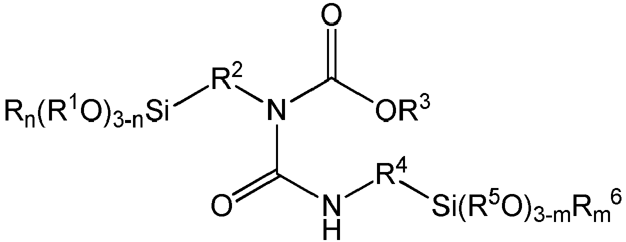 dans laquelle R, R¹-R⁶ représentent, indépendamment les uns des autres, des radicaux hydrocarbonés identiques ou différents, comprenant 1-8 atomes de carbone, ceux-ci pouvant être ramifiés ou cycliques, et m et n valent 0.

2. Monoallophanates de formule 1 selon la revendication 1, R¹ et R⁵ signifiant, indépendamment l'un de l'autre, méthyle ou éthyle.

3. Monoallophanates de formule 1 selon la revendication 1, R² et R⁴ signifiant, indépendamment l'un de l'autre, méthyle ou propyle.

4. Monoallophanates de formule 1 selon la revendication 1, R¹ = R⁵ et R² = R⁴.

5. Monoallophanates de formule 1 selon la revendication 1, m et n valant 0, R¹ et R⁵ signifiant méthyle ou éthyle et R² et R⁴ signifiant méthyle ou propyle.

6. Monoallophanates de formule 1 selon la revendication 1, m et n valant 0, R¹ = R⁵ = méthyle et R² = R⁴ = propyle.

7. Procédé pour la préparation des monoallophanates selon les revendications 1-6 par réaction
A) d'un isocyanate contenant des groupes alcoxysilane avec
B) un mono-uréthane contenant des groupes alcoxysilane,
à des températures dans la plage de 20 à 220°C.

8. Procédé pour la préparation des monoallophanates selon la revendication 7, les isocyanates A) contenant des groupes alcoxysilane présentant la formule 2 :
R⁶m (OR⁵)₃₋ₘSi-R⁴-NCO
dans laquelle R⁶, R⁵ et R⁴ représentent, indépendamment les uns des autres, des radicaux hydrocarbonés identiques ou différents, comprenant 1-8 atomes de carbone, ceux-ci pouvant être ramifiés ou cycliques, et m vaut 0.

9. Procédé pour la préparation des monoallophanates selon la revendication 8, m valant 0 et R⁴ signifiant méthyle ou propyle et R⁵ signifiant méthyle ou éthyle.

10. Procédé pour la préparation des monoallophanates selon la revendication 8, de l'isocyanatopropyltriméthoxysilane étant utilisé.

11. Procédé pour la préparation des monoallophanates selon la revendication 7, les mono-uréthanes B) contenant des groupes alcoxysilane présentant la formule 3 :
Rₙ (OR¹)₃₋ₙSi-R²-NH-(C=O)-OR³
dans laquelle R, R¹, R² et R³ représentent, indépendamment les uns des autres, des radicaux hydrocarbonés identiques ou différents, comprenant 1-8 atomes de carbone, ceux-ci pouvant être ramifiés ou cycliques, et n vaut 0.

12. Procédé pour la préparation des monoallophanates selon la revendication 11, n valant 0 et R² signifiant méthyle ou propyle, R¹ signifiant méthyle ou éthyle et R³ = R¹.

13. Procédé pour la préparation des monoallophanates selon la revendication 11, du N-triméthoxysilylpropylméthylcarbamate étant utilisé.

14. Utilisation des monoallophanates selon les revendications 1-6 dans des compositions de laque et des compositions adhésives.
